# EUROPÄISCHE PATENTSCHRIFT

(11) **EP 0 447 734 B1**
(45) Veröffentlichungstag und Bekanntmachung des Hinweises auf die Patenterteilung: **06.10.1993**
(21) Anmeldenummer: 90810956.4
(22) Anmeldetag: 06.12.1990
(51) Int. Cl.: A61F 2/30

(54) **Knochenzementfreie Femurkopfprothese**
Femoral head prosthesis for cementless affixation
Prothèse de tête du fémure destinée à être fixée sans ciment

(30) Priorität: 13.03.1990 CH 798/90
(43) Veröffentlichungstag der Anmeldung: 25.09.1991
(73) Patentinhaber: SULZER Medizinaltechnik AG, 8404 Winterthur (CH); PROTEK AG, 3001 Bern (CH)
(72) Erfinder: Marchetti, Pier Giorgio, Prof. Dr.-med., I-40000 Bologna (IT); Willi, Roland, CH-8543 Stadel (CH); Koch, Rudolf, CH-8267 Berlingen (CH)
(74) Vertreter: Hammer, Bruno, Dr.

(56) Entgegenhaltungen:
- EP-A- 0 196 258
- EP-A- 0 217 034
- EP-A- 0 273 871

## Beschreibung

Die Erfindung betrifft eine knochenzementfreie Femurkopfprothese, die jeweils aus einem einheitlichen Schaftkörper und auswechselbaren Stützkörpern besteht, die eine individuelle Anpassung eines Schaftes an eine bestehende Knochenaushöhlung ermöglichen. Bausätze dieser Art sind gezeigt in CH-PS 671 689, EP-A-0 217 034 und CH-Patentgesuch Nr. 0385/88-3. Sie erlauben es, eine Auswahl der Stützkörper während der Operation vorzunehmen. Eine Femurkopfprothese gemäß den Merkmalen des Oberbegriffs des Anspruchs 1 ist aus der EP-A-0 217 034 bekannt.

Allgemein besteht bei den knochenzementfreien Femurkopfprothesen die Aufgabe darin, eine Primärverankerung mit viel Kontaktfläche zum Knochengewebe zu schaffen, ohne dass das später einwachsende Knochengewebe in bestimmten Zonen durch zu grosse Scherbeanspruchung zwischen Prothese und Knochengewebe in seiner Blutversorgung unterbrochen wird und abstirbt.

Diesem Umstand trägt die Erfindung Rechnung. Sie hat die Aufgabe, ein späteres Setzen vom Prothesenschaft möglichst klein zu halten. Gemäss der Erfindung wird die Aufgabe dadurch gelöst, dass die Backen jeweils einen in seiner Grösse auswählbaren, lateral und ventral oder lateral und dorsal vorstehenden Wulst beim Übergang von ventral oder dorsal nach lateral aufweisen, welcher sich von lateral zu medial und von proximal zu distal konisch verjüngend der Schaftoberfläche angleicht.

Der Vorteil der Erfindung ist darin zu sehen, dass der Schaft am proximalen Schaftende, bezogen auf seine Längsachse, allseitig durch eine zusammenhängende, sich zur Schaftspitze hin konisch verjüngende Fläche zentriert ist, die gleichzeitig eine gleichmässige Verteilung der Hauptlast gewährleistet und in der Längsachse wirkende Drehmomente übernehmen kann. Als weiteres ist diese konische Fläche entsprechend den vorgefundenen Verhältnissen am Femurknochen während der Operation anpassbar. Die Ansprüche 1 bis 8 beziehen sich auf vorteilhafte Weiterbildungen der Erfindung.

Im folgenden wird die Erfindung anhand von einem Ausführungsbeispiel beschrieben. Es zeigen:

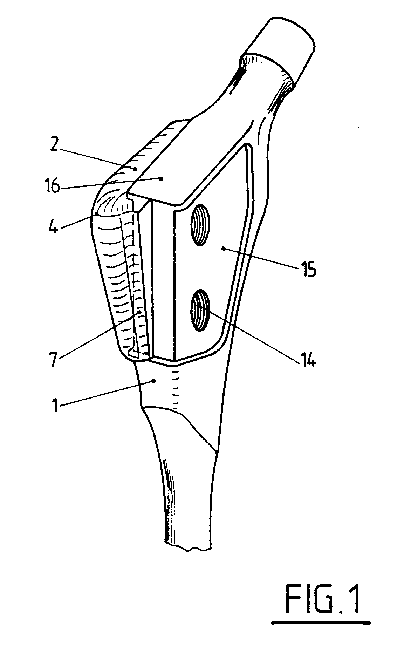

- Fig. 1: Die Ansicht von einem Schaftkörper, bei dem eine Backe mit Wulst bereits aufgesetzt ist;

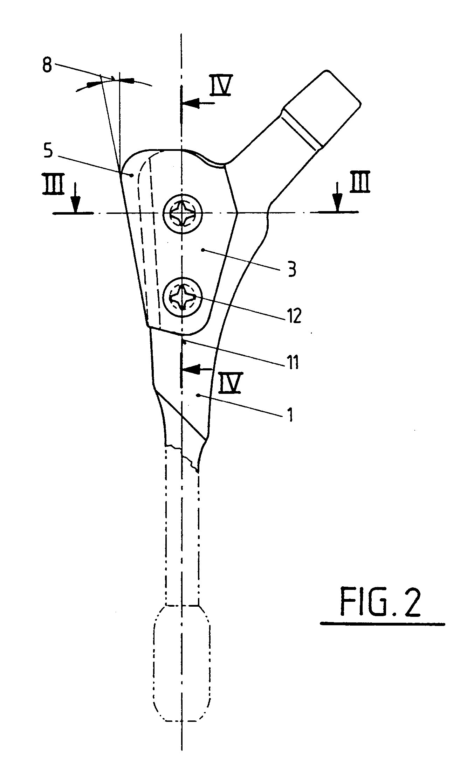

- Fig. 2: die Seitenansicht eines Schaftkörpers mit aufgesetzten Backen;

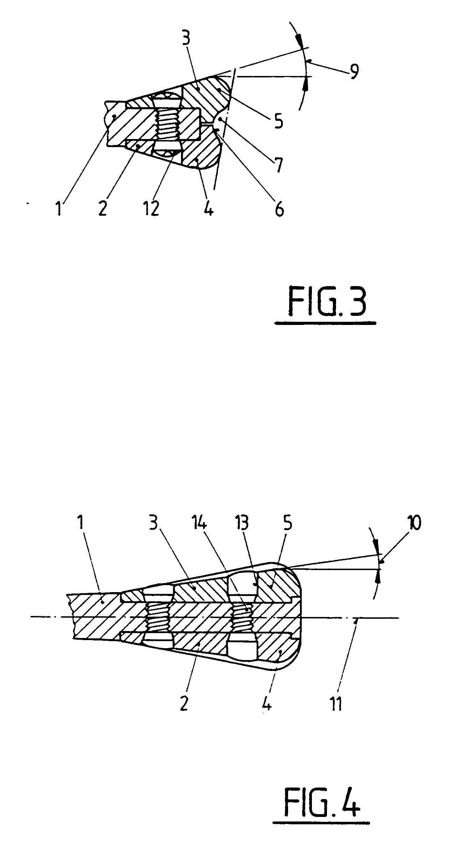

- Fig. 3: einen Schnitt quer zur Längsachse des Schaftes auf der Höhe der aufgesetzten Backen nach Fig. 2;
- Fig. 4: einen Schnitt in der Längsachse des Schaftes auf der Höhe der aufgesetzten Backen nach Fig. 2, jedoch ohne Befestigungsschrauben.

In den Figuren ist eine knochenzementfreie Femurkopfprothese gezeigt, die aus jeweils einem einheitlichen Schaftkörper 1 und auswechselbaren Stützkörpern besteht. Typische Stützkörper sind z.B. auswechselbare Schaftspitzen. Erfindungsgemäss sind am proximalen Schaftende 16 ventral und dorsal auswechselbare Backen 2, 3 befestigt, die das Schaftende 16 lateral umgreifen und einen in seiner Grösse auswählbaren Wulst 4, 5 aufweisen. In Richtung der Schaftachse 11 betrachet, stehen diese Wulste 4, 5 am meisten beim Uebergang von dorsal oder ventral nach lateral vor und verjüngen sich konisch zur Schaftoberfläche auslaufend einmal auf der dorsalen und ventralen Seite von lateral nach medial und zum anderen auf der lateralen, dorsalen und ventralen Seite von proximal nach distal. Lateral nehmen die Wulste 4, 5 bis zu einer Trennstelle 6 der Backen 2, 3 ab und bilden gemeinsam eine Rinne 7. Diese Rinne 7 stellt eine zusätzliche Drehsicherung gegen Momente in der Schaftachse 11 dar, da die passende Schulter beim Raspeln der Hohlform im Knochengewebe stehen bleibt. Die Knochenraspeln und die Wülste 4, 5 der Backen 2, 3 sind stufenweise aufeinander abgestimmt, indem grössere Wülste 4, 5 auch grösseren Verjüngungswinkeln entsprechen. So entspricht der mittlere Verjüngungswinkel 9 von lateral nach medial etwa 15° und kann je nach vorgefundenem Femurknochen in den Grenzen von 0° bis 25° schwanken. Der mittlere Verjüngungswinkel 8 auf der lateralen Seite der Backen 2, 3 von proximal nach distal entspricht einem Winkel von 10° und kann je nach vorgefundenem Femurknochen in den Grenzen von 0° bis 20° schwanken. Auf der ventralen und dorsalen Seite verjüngen sich die Backen 2, 3 mit einem Winkel 10 von proximal nach ventral, der im Mittel etwa 9° entspricht und je nach vorgefundenen Verhältnissen zwischen 0° und 15° schwanken kann. Durch die stufenweise und voneinander unabhängige Anpassbarkeit der beiden Backen 2, 3 können relativ grosse konische Verjüngungswinkel 8, 9, 10 erzeugt werden, die für eine gute Verteilung der Hauptlast bei geringen spezifischen Belastungen auf das einwachsende Gewebe sorgen und einem späteren Absenken des Schaftes 1 entgegenstehen. Die Backen 2, 3 sind an ihrer Aussenseite mit einer Struktur versehen, die das Einwachsen von Knochengewebe fördert. Zur Befestigung der Backen mit Schrauben 12 enthalten diese Absenkungen 13 und der Schaft 1 Gewindelöcher 14. Die Befestigungsflächen 15 für die Backen 2, 3 sind in den Schaft 1 eingelassen, damit die Backen 2, 3 im konisch auslaufenden Bereich noch eine vernünftige Dicke haben und damit die Schrauben 12 weniger Kräfte von den Backen 2, 3 in Richtung der Schaftachse 11 übernehmen müssen. Die auf die Stufung der Backen 2, 3 abgestimmten Bearbeitungsraspeln sind während der Bearbeitung geführt, um ein möglichst genaues Anliegen des während der Operation zusammengestellten Schaftes 1 mit seinen konischen Partien zu ermöglichen.

## Patentansprüche

1. Knochenzementfreie Femurkopfprothese bestehend aus jeweils einem einheitlichen Schaftkörper (1) und auswechselbaren Stützkörpern, die eine individuelle Anpassung des Schaftes an eine bestehende Knochenaushöhlung ermöglichen, wobei am proximalen Schaftende (16) ventral und dorsal auswechselbare Backen (2, 3) befestigt sind, dadurch gekennzeichnet, dass die Backen (2,3) jeweils einen in seiner Grösse auswählbaren, lateral und ventral oder lateral und dorsal vorstehenden Wulst (4, 5) beim Übergang von ventral oder dorsal nach lateral aufweisen, welcher sich von lateral zu medial und von proximal zu distal konisch verjüngend der Schaftoberfläche angleicht.

2. Bausatz mit mehreren Schaftgrössen nach Anspruch 1, dadurch gekennzeichnet, dass die Wulste (4, 5) der Backen (2, 3) lateral zu einer Trennstelle (6) der beiden Backen zurückschwingen und gemeinsam eine Rinne (7) von proximal nach distal bilden.

3. Bausatz mit mehreren Schaftgrössen nach Anspruch 1 und 2, dadurch gekennzeichnet, dass die vom Schaft (1) abgekehrte Oberfläche der Backen (2, 3) eine Struktur mit Vorsprüngen und Rücksprüngen aufweist.

4. Bausatz nach einem der Ansprüche 1 bis 3, dadurch gekennzeichnet, dass die Backen (2, 3) teilweise in den Schaft (1) eingelassen sind.

5. Bausatz nach einem der Ansprüche 1 bis 3, dadurch gekennzeichnet, dass die Wulste (4, 5) der Backen (2, 3) sich lateral mit einem Winkel (8) zwischen 0 und 20° von proximal nach distal verjüngen.

6. Bausatz nach einem der Ansprüche 1 bis 3, dadurch gekennzeichnet, dass die Wulste (4, 5) der Backen (2, 3) sich dorsal und ventral mit einem Winkel (9) zwischen 0 und 25° von lateral nach medial verjüngen.

7. Bausatz nach einem der Ansprüche 1 bis 3, dadurch gekennzeichnet, dass die Wulste (4, 5) der Backen (2, 3) sich dorsal und ventral mit einem Winkel (10) zwischen 0 und 15° von proximal nach distal verjüngen.

8. Bausatz nach einem der Ansprüche 1 bis 7, dadurch gekennzeichnet, dass zu den verschiedenen Formen der Backen (2, 3) modulare Raspeln bestehen, die eine geführte Bearbeitungsbewegung beim Erweitern einer Knochenaushöhlung ermöglichen.

## Claims

1. A femur-head prosthesis free of bone cement and consisting in a given case of one uniform shank body (1) and replaceable supporting bodies which enable individual adaptation of the shank to an existing bone cavity, there being ventrally and dorsally replaceable cheeks (2, 3) fastened to the proximal end (16) of the shank, characterized in that in transition from ventral or dorsal to lateral each cheek (2, 3) exhibits a bulge (4, 5) which projects laterally and ventrally or laterally and dorsally and may be chosen for size and is adapted to the surface of the shank by tapering in conically from lateral to medial and from proximal to distal.

2. A kit having a number of shank sizes as in Claim 1, characterized in that the bulges (4, 5) on the cheeks (2, 3) curl back laterally to a line (6) of separation of the two cheeks and together form a groove (7) from proximal to distal.

3. A kit having a number of shank sizes as in Claims 1 and 2, characterized in that the surface of the cheeks (2, 3) remote from the shank (1) exhibits a structure with projections and recesses.

4. A kit as in one of the Claims 1 to 3, characterized in that the cheeks (2, 3) are partially let into the shank (1).

5. A kit as in one of the Claims 1 to 3, characterized in that the bulges (4, 5) on the cheeks (2, 3) taper in laterally from proximal to distal at an angle (8) between 0 and 20°.

6. A kit as in one of the Claims 1 to 3, characterized in that the bulges (4, 5) on the cheeks (2, 3) taper in dorsally and ventrally from lateral to medial at an angle (9) between 0 and 25°.

7. A kit as in one of the Claims 1 to 3, characterized in that the bulges (4, 5) on the cheeks (2, 3) taper in dorsally and ventrally from proximal to distal at an angle (10) between 0 and 15°.

8. A kit as in one of the Claims 1 to 7, characterized in that for the different shapes of the cheeks (2, 3) modular rasps exist, which enable a guided machining motion in the widening of a bone cavity.

## Revendications

1. Prothèse de tête de fémur à fixer sans ciment pour os, constituée d'un corps de tige (1) standard et de corps d'appui échangeables qui permettent une adaptation individuelle de la tige à une cavité osseuse existante, des joues (2, 3) échangeables étant fixées ventralement et dorsalement sur l'extrémité (16) proximale de la tige, caractérisée en ce que les joues (2, 3) comportent, dans la zone de transition entre la partie ventrale ou dorsale et la partie latérale, un bourrelet (4, 5), de taille à sélectionner, faisant saillie latéralement et ventralement ou latéralement et dorsalement et qui, se rétrécissant coniquement de la partie latérale vers la partie médiale et de la partie proximale vers la partie distale, s'adapte à la surface de la tige.

2. Jeu de plusieurs tailles de tige selon la revendication 1, caractérisé en ce que les bourrelets (4, 5) des joues (2, 3) se rétrécissent latéralement vers un point de séparation (6) des deux joues et forment conjointement une gorge (7) s'étendant de la partie proximale à la partie distale.

3. Jeu de plusieurs tailles de tige selon les revendications 1 et 2, caractérisé en ce que la surface, opposée à la tige (1), des joues (2, 3) présente une structure avec des parties saillantes et des parties en retrait.

4. Jeu selon l'une des revendications 1 à 3, caractérisé en ce que les joues (2, 3) sont partiellement encastrées dans la tige (1).

5. Jeu selon l'une des revendications 1 à 3, caractérisé en ce que les bourrelets (4, 5) des joues (2, 3) se rétrécissent latéralement suivant un angle (8) compris entre 0 et 20°, de la partie proximale à la partie distale.

6. Jeu selon l'une des revendications 1 à 3, caractérisé en ce que les bourrelets (4, 5) des joues (2, 3) se rétrécissent dorsalement et ventralement sous un angle (9) compris entre 0 et 25°, de la partie latérale à la partie médiale.

7. Jeu selon l'une des revendications 1 à 3, caractérisé en ce que les bourrelets (4, 5) des joues (2, 3) se rétrécissent dorsalement et ventralement, sous un angle (10) compris entre 0 et 15°, de la partie proximale vers la partie distale.

8. Jeu selon l'une des revendications 1 à 7, caractérisé en ce qu'il existe pour les différentes formes de joues (2, 3), des râpes modulaires qui permettent un déplacement d'usinage guidé lors de l'élargissement d'une cavité osseuse.
